Europäisches Patentamt

European Patent Office

Office européen des brevets

(19)

(11) Veröffentlichungsnummer : **0 332 571 B1**

# (12) EUROPÄISCHE PATENTSCHRIFT

(45) Veröffentlichungstag der Patentschrift :
28.08.91 Patentblatt 91/35

(51) Int. Cl.⁵ : **A61F 2/36**

(21) Anmeldenummer : **89810044.1**

(22) Anmeldetag : **19.01.89**

(54) Verankerungsschaft für eine Femurkopfprothese.

(30) Priorität : 26.02.88 CH 720/88

(43) Veröffentlichungstag der Anmeldung :
13.09.89 Patentblatt 89/37

(45) Bekanntmachung des Hinweises auf die
Patenterteilung :
28.08.91 Patentblatt 91/35

(84) Benannte Vertragsstaaten :
AT DE FR GB IT

(56) Entgegenhaltungen :
DE-A- 2 627 569
DE-U- 8 811 758
FR-A- 2 578 738

(73) Patentinhaber : GEBRÜDER SULZER
AKTIENGESELLSCHAFT
Zürcherstrasse 9
CH-8401 Winterthur (CH)
Patentinhaber : Protek AG
Stadtbachstrasse 64
CH-3001 Bern (CH)

(72) Erfinder : Giacometti, Roberto, Prof.Dr.-med.
Centro Ortopedico R. Galeazzi Via R. Galeazzi
4
I-20161 Milano (IT)

EP 0 332 571 B1

## Beschreibung

Die Erfindung betrifft einen Verankerungsschat für eine Femurkopfprothese, dessen distaler Bereich einen runden Querschnitt aufweist und über eine gewisse Länge hohl ausgebildet ist, wobei die Hohlraumrwand mit einem Längsschlitz versehen ist, welcher nach medial gerichtet ist, d.h. mit der Prothesenhalsachse in einer Ebene liegt.

Ein Prothesenschaft der vorstehend geschilderten Art ist beispielsweise bekannt aus der EP-A-222236 ; bei diesem Schaft ist das distale Ende mit einer zentralen Bohrung in Richtung der Längsachse versehen, wobei zusätzlich die die Bohrung umhüllende Wand durch zwei senkrecht zueinander angeordnet Schlitze in vier einzelne Segmente unterteilt ist. Bohrung und Schlitze dienen dazu, Verformungen, insbesondere ein Zusammendrücken des distalen Endes an Unebenheiten und Verengungen des Operationshohlraumes zu ermöglichen.

Infolge der Biegebelastung, die über den Gelenkkopf auf den Schaft ausgeübt wird, wird der distale Bereich des Schaftes in Richtung lateral gepresst. Besonders bei Reoperationsprothesen, bei denen die Schäfte relativ lang sind und weit in den Femurknochen hineinreichen, hat es sich gezeigt, dass die allseitig gleichen relativ dünnen Wände im distalen Bereich den erwähnten Belastungen in lateraler Richtung gegenüber zu nachgiebig sind, so dass es im proximalen Bereich des Schaftes zu unerwünscht grossen Mikrobewegungen gegenüber dem Knochen kommt. Da andererseits die relativ langen Reoperationsschäfte in fortschreitend enger werdende Abschnitte des Femurs vordringen, ist jedoch eine erhöhte Nachgiebigkeit im distalen Endbereich erstrebenswert. Aufgabe der Erfindung ist es daher, einen, vor allem als Schaft für eine Reoperationsprothese dienenden Verankerungsschaft zu schaffen, der gegenüber den in lateraler Richtung wirkenden Belastungskräften unnachgiebig ist, jedoch gegen sein distales Ende hin eine zunehmende Nachgiebigkeit hat.

Diese Aufgabe wird mit der Erfindung dadurch gelöst, dass der Hohlraum von distal nach proximal fortschreitend nach medial versetzt ist und eine abnehmende Querschnittsfläche hat.

Die von distal nach proximal fortschreitende Versetzung des Hohlraumes nach medial und die in gleicher Richtung erfolgende Verringerung seiner Querschnittsfläche bewirken, dass die laterale Wandstärke — besonders in den nach proximal gelegenen Teilen des distalen Bereiches — relativ gross bleibt und damit eine hohe Steifigkeit behält, die in Richtung auf das distale Ende stufenweise oder kontinuierlich kleiner wird. Trotz der Steifigkeit im oberen Teil des distalen Schaftbereiches ist die geforderte Zusammendrückbarkeit in ausreichendem Masse durch den nach medial gerichteten Längsschlitz und den damit in Verbindung stehenden, ebenfalls nach medial orientierten Hohlraum gewährleistet. Ein weiterer Vorteil der neuen Konstruktion ist darin zu sehen, dass sich der Hohlraum im Laufe der Zeit mit einwachsendem Gewebe füllt, welches durch den Schlitz hindurch ernährt und lebensfähig gehalten wird.

Mit Vorteil erstrecken sich Hohlraum und Längsschlitz zumindestens nahezu bis zur Mitte der Schafthöhe, die als der Abstand zwischen dem distalen Schaftende und einer proximalen Schulter gemessen wird, mit der die laterale Schmalseite in den Prothesenhals übergeht.

Bei der Umsetzung des Grundgedankes der Erfindung in die Praxis hat es sich als zweckmässig erwiesen, wenn der Hohlraum aus stufenweise abgesetzten Bohrungen besteht, deren Durchmesser sich von Stufe zu Stufe von distal nach proximal verringern, wobei die Achsen der Bohrungen in gleicher Richtung fortschreitend nach medial versetzt sind.

Im folgenden wird die Erfindung anhand eines Ausführungsbeispeils im Zusammenhang mit der Zeichnung näher erläutert.

Fig. 1        zeigt teilweise im Schnitt eine ventral/dorsal gesehene Ansicht eines Verankerungsschaftes für eine Reoperationsprothese ; die

Fig. 2a bis 2f      sind die entsprechenden Schnitt A-A bis F-F von Fig. 1.

Der gerade Schaft 1 ist seinem distalen Bereich bis etwa zur Ebene des Schnittes B-B im Querschnitt kreisförmig, während der proximale Teil eine elliptische oder ovale Form hat. Die laterale Schmalseite 2 des proximalen Querschnittes ist zunächst zur Längsachse hin leicht geneigt und geht über eine horizontale Schulter 4 in den Prothesenhals 5 über. An diesem ist ein konischer Zapfen 6 angesetzt, der den nicht gezeigten Gelenkkopf aufnimmt.

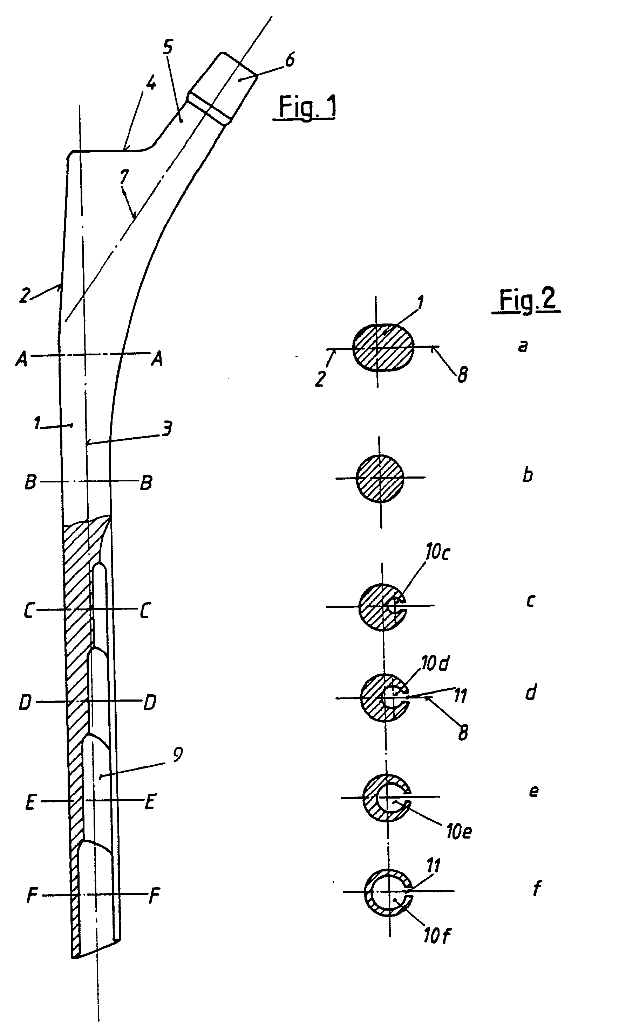

Medial führt ein bogenförmiger Uebergang aus dem geraden distalen Bereich stufenlos in den Prothesenhals 5. Dessen Achse 7 definiert mit der Längsachse 3 eine Ebene 8 (Fig. 2a).

Vom Ende her ist im distalen zylindrischen Bereich des Schaftes 1 ein Hohlraum 9 geschaffen, der aus einer Anzahl Bohrungen 10c bis 10f mit unterschiedlichen Durchmessern besteht ; diese Bohrungen, deren Durchmesser von proximal nach distal zunehmen, verlaufen parallel zur Längsachse 3. Bei einem Teil 10c bis 10e von ihnen sind die Achsen jedoch gegen die Längsachse in der Ebene 8 nach medial versetzt. In dieser Ebene 8 ist weiterhin die Wand jeder Bohrung 10c bis 10f von einem Längsschlitz 11 unterbrochen. Beidseits dieses Schlitzes 11 wird somit ein Hohlraum 9 von dünnwandigen verformbaren Lappen begrenzt, deren Verformbarkeit mit steigendem Volumen des Hohlraumes 9 nach distal zunimmt.

## Patentansprüche

1. Verankerungsschaft (1) für eine Femurkopfprothese, dessen distaler Bereich einen runden Querschnitt aufweist und über eine gewisse Länge hohl ausgebildet ist, wobei die Hohlraumwand mit einem Längsschlitz (11) versehen ist, welcher nach medial gerichtet ist, d.h. mit der Prothesenhalsachse (7) in einer Ebene liegt, **dadurch gekennzeichnet, dass** der Hohlraum (9) von distal nach proximal fortschreitend nach medial versetzt ist und eine abnehmende Querschnittsfläche hat.

2. Verankerungsschaft nach Anspruch 1, **dadurch gekennzeichnet, dass** der Hohlraum (9) aus stufenweise abgesetzten Bohrungen (10c-10f) besteht, deren Durchmesser sich von Stufe zu Stufe von distal nach proximal verringern, wobei die Achsen der Bohrungen (10c-10f) in der gleichen Richtung fortschreitend nach medial versetzt sind.

3. Verankerungsschaft nach Anspruch 1 oder 2, **dadurch gekennzeichnet, dass** der Hohlraum (9) mindestens annähernd bis zur Mitte der Schafthöhe reicht, die vom distalen Ende bis zu einer den Übergang zum Prothesenhals (5) bildenden Schulter gemessen ist.

## Claims

1. A fixing stem (1) for a femoral head prosthesis whose distal zone is of round cross-section and is hollow over some of its length, the cavity wall being formed with a slot (11) which is directed medially—i.e., is coplanar with the prosthesis neck axis (7)—, characterised in that the cavity (9) has an increasing medial offset distally to proximally and a decreasing cross-sectional area.

2. A fixing stem according to claim 1, characterised in that the cavity (9) is in the form of stepped bores (10c-10f) whose diameters decrease from step to step distally to proximally, the axes of the bores (10c-10f) being progressively offset medially in the same direction.

3. A fixing stem according to claim 1 or 2, characterised in that the cavity (9) extends at least substantially as far as the centre of stem height, the same being measured from the distal end as far as a shoulder forming the transition to the prosthesis neck (5).

## Revendications

1. Tige d'ancrage (1) pour une prothèse de tête de fémur, dont la région distale présente une section circulaire et qui est réalisée creuse sur une certaine longueur, la paroi de la cavité présentant une fente longitudinale (11) qui est dirigée vers l'intérieur, c'est-à-dire qu'elle se trouve dans un même plan avec l'axe (7) du col de prothèse, caractérisée en ce que la cavité (9) est décalée progressivement vers l'intérieur de la partie distale vers la partie proximale et a une aire de section transversale décroissante.

2. Tige d'ancrage suivant la revendication 1, caractérisée en ce que la cavité (9) est constituée de trous (10c-10f) décalés par degrés, dont le diamètre décroît de degré en degré de la partie distale vers la partie proximale, les axes des trous (10c-10f) étant progressivement décalés vers l'intérieur dans la même direction.

3. Tige d'ancrage suivant la revendication 1 ou 2, caractérisée en ce que la cavité (9) s'étend au moins approximativement jusqu'au milieu de la hauteur de la tige, qui est mesurée de l'extrémité distale jusqu'à un épaulement formant la transition avec le col (5) de la prothèse.

Fig. 1

Fig. 2